# EUROPEAN PATENT APPLICATION

(11) **EP 1 154 014 A1**
(43) Date of publication of application: **14.11.2001**
(21) Application number: 99961282.3
(22) Date of filing: 08.12.1999
(51) Int. Cl.: C12M 1/34, G01N 33/50, C12Q 1/18

(54) **PAPER PIECES TRANSFER METHOD AND AUTOMATIC PAPER PIECES TRANSFER DEVICE**

(30) Priority: 17.02.1999 JP 3896299; 17.02.1999 JP 3896399; 22.02.1999 JP 4336699
(71) Applicant: Nittetsu Mining Co., Ltd., Tokyo 100-0005 (JP); SANKYO COMPANY LIMITED, Chuo-ku, Tokyo 103-0023 (JP)
(72) Inventor: INABA, Kazuhiro, Nittetsu Mining Co, Ltd, Nishitama-gun, Tokyo 190-0182 (JP); SHIRATORI, Mamoru Sankyo Compagny, Ltd., Tokyo 140-0005 (JP); ICHIKAWA, Masato, Sankyo Compagny, Ltd., Tokyo 140-0005 (JP)
(74) Representative: Grünecker, Kinkeldey, Stockmair & Schwanhäusser Anwaltssozietät
(86) International application number: JP9906884
(87) International publication number: WO0049131

(57) **Abstract**

A plurality of slips (17) supplied in a lump to the input part (15) are branched into two rows through a diversion part (27), and the slips (17) are set forward to array in two rows by the array straight advance feeder (25) connected to the respective branch channels (23). On the other hand, the branch hose on the side opened to the atmosphere is blocked, and the branch hose on the side connected to the pump (51) is opened, thereby causing the slips (17) on the array straight advance feeder (25) to be held by adsorbing by the air inhaled from the suction nozzle (43) and moved to the culture medium side. Thereafter, the branch hose on the connection side to the pump (51) is closed and the hose on the side opened to the atmosphere is opened to release the suction-holding of the slips (17) by the suction nozzle (43) and transpose the slips (17) to the predetermined position of the agar medium. This suction nozzle (43) constitutes the suction port of the suction nozzle (43) so as to freely advance and recede to the slips (17) on the slip supply side, and to the culture medium on the culture medium side.

## Description

### Technical Field

The invention relates to a slip transposing method and an automatic slip transposing apparatus for taking out the predetermined number of slips from the plural slips charged in a lump and placing such slips on the predetermined positions of the culture medium.

### Background Art

The method of knowing the effectiveness and effect of the antibiotics includes one by measuring the concentration of the antibiotics remaining in the blood, urea, and organs of the animal administered with the antibiotics. As one of the methods of such measuring method, there is a disk method in which there is a slip placed on a agar plate medium mixed with bacteria, (hereinafter to be called alias "disk") of about 6 mm in diameter made by permeating a tissue extract liquid or a sampled body fluid diluted to an moderate concentration, and thereafter, the agar plate medium is cultured, then the agar plate medium is cultured and caused to inhibit the growth of bacteria in proportion to the concentration of the antibiotics permeated into the disk, and the size of the area (arrest circle) is determined and the effect of the antibiotics is known.

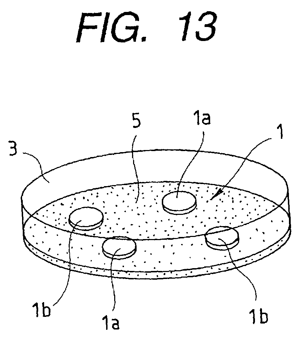

According to this disk method, firstly, body fluid of the animal/patient administered with antibiotics is sampled and have the liquid diluted or extracted from the tissue permeate into the disk. The disk in which the reference liquid or the test body liquid is permeated is lightly placed on a blotting paper to eliminate extra moisture. Next, as shown in Fig. 13, the disk 1 provided with permeation is placed one by one on a predetermined position on the agar plate 5 formed on the laboratory dish 3 (normally, four places of 36 mm X 36 mm). In this case, there are placed a couple of the disks 1a, 1a permeated with the standard liquid at one opposed angle corners of the square and the other couple 1b, 1b permeated with the test body at the other opposed angle corners, i.e., four in total. As those disks require to be treated nearly simultaneously, the work is carried out by two testers.

The agar plate 5 provided with four disks 1 at its diagonal corners is cultured under the predetermined conditions. After culture, the size of the inhibition circle formed by inhibition of growth of the bacteria in proportion to the concentration of the antibiotics is measured and the concentration of the antibiotics of the test body is computed. By this step, it has been possible to know the effectiveness and effect of the antibiotics.

In the conventional disk method for the purpose of knowing the effectiveness and effect of the antibiotics, the disk had been placed by the manual work by the tester. Accordingly, there were problems such that it was not possible to expect energy saving, work duration was long, and yet irregularity occurred in the position of transposition of the disk. In view of this status, it has been desired to have the apparatus developed in which the slip can be automatically transposed.

However, there are various problems that have to be solved for the realization of the automatic slip transposing apparatus as follows.

Namely, as the culture medium surface on which the disk is to be transposed has moisture, the moisture absorption degree from the culture medium surface differs depending on the time of transposition, involving the risk of affecting the measurement results. Therefore, it was required to place four disks simultaneously on the opposite angles of a square. Further, as the disks are light slips, it was difficult to array a plurality of light slips supplied in a lump one by one without overlap.

Furthermore, it was necessary to hold with assurance the air permeable disks and transpose with assurance the disks in the held condition to the predetermined position on the agar medium. And, it was necessary to obtain a transposing apparatus of high reliability free of choking in a simple structure without necessitating the complicated and precise valve structure. Further, in order to carry out smooth transposing work continuously, detection of erroneous holding of the disk is indispensable.

Further, as the disk is required to be transposed onto a weak agar medium, in transposing the disk by the disk holding means, the disk holding means require to be position-controlled in high precision. Due to the irregularity of the transfer structure of the disk holding means or the agar medium surface, in case of any error caused to the position precision, there is a danger for the excessive pressure beyond necessity to be exerted to the culture medium to cause speck to the culture medium.

The present invention has been made in reflection of the above-mentioned condition. The primary object of the invention is to provide a slip transposing method and an automatic slip transposing apparatus wherein the predetermined number of slips can be taken out from a plurality of slips supplied in bulk, and the respective slips can be automatically transposed in the predetermined positions of the culture medium.

The second object of the invention is to provide a slip transposing method and an automatic slip transposing apparatus with which assured holding and transposing of the disk can be made, constitution can be made in a simple structure free from choking, and detection of the mistake of holding the disk is possible.

The third object of the invention is to provide a slip transposing method and an automatic slip transposing apparatus wherein transposing can be performed without causing speck to the culture medium even in case of the position control in relatively low precision.

### Means for solving the problems

In order to attain the objects, the slip transposing method of the present invention includes branching in two rows and nearly horizontally arraying the slips sent out from the slip input portion in which a plurality of slips are supplied, and on the other hand, suction-holding the predetermined number of the arrayed slips on each row to transpose the slips onto the respective predetermined positions on the culture medium.

According to this slip transposing method, it is possible to transpose the slips simultaneously onto the predetermined respective positions on the culture medium by the slip suction nozzles which suction-hold the predetermined numbers of slips sent out from the slip input portion.

Another method of transposing the slips of the invention includes sending out a plurality of slips sequentially to the discharge channel from the slip input portion that supplies a plurality of slips, dividing the slips into two rows in approximately evenly by the step differences provided halfway in the exhaust channel, and further stopping the slips at the tip, thereby arraying the slips in forward order in two rows, and on the other hand, taking out a predetermined number of the arrayed slips to place in the predetermined positions of the culture medium.

According to this slip transposing method, because a couple of slips in the same position are taken out from the respective rows in which the slips are arrayed, it becomes possible for the four slips to be arranged on the diagonal lines of a square. By this device, it becomes possible for the four slips on the diagonal line that had so far been transposed manually to be transposed simply and in high positional precision.

Furthermore, the slip transposing method according to the present invention includes, communicably connecting an end of the elastic hose to a suction nozzle for slip, dividing the other end of the elastic hose into two ways, connecting one of the branched part to the pump and opening the other part to the atmosphere, blocking the hose on the side opened to the atmosphere to render the inside of the hose to a negative pressure by the pump and suction-holding the slip from the supply part of the slip with the suction nozzle, blocking the hose on the side connected to the pump, and opening the hose on the side opened to the atmosphere, thus rendering the inside of the hose to the atmospheric pressure and releasing the suction-holding of the slip by the nozzle to transpose the slip from the slip supply portion to the culture medium.

According to this slip transposing method, the open side on the other end of the hose is blocked to make the hose inside negative pressure, and the slip is held by suction by the atmosphere sucked by the pump from the suction nozzle. On the other hand, the other end of the hose on the pump side is blocked and the opened side of the other end of the hose is opened, by which the atmospheric pressure is introduced into the hose, and under impact of the introduced air, the slip that had been held by suction is surely separated without being caught by the tip of the suction nozzle and placed on the agar medium.

As other method, the air pressure in the hose may be detected by the air pressure detection sensor in the hose, and if the pressure is higher than the predetermined value, the suction may be rescinded.

In this case, when the suction nozzle has failed to adsorb the slip, the air pressure detection sensor detects that the pressure in the hose becomes more than the preset predetermined value, and it becomes possible to detect the holding mistake of the slip by the suction nozzle. In case of the air-permeable slip, if the pressure difference by the suction resistance occurs on the detectable level at the time of the suction holding, detection of the holding mistake is possible.

Further, the slip transposing method of the invention is a slip transposing method of taking out a predetermined number of slips out of the plural slips in the slip supply portion and transposing the slips to the respective predetermined positions on the culture medium, characterized in that, in holding by suction the slip on the slip supply portion from the suction port at the tip of the suction nozzle for sucking the atmosphere, and in placing the slip on the culture medium by moving it from the slip supply portion side to the culture medium side, the forcing means provided on the suction nozzle and forcing in the direction of the suction hole at the tip of the nozzle is compressed under the predetermined pressure force from the suction port at the tip at the time when the suction nozzle is lowered.

According to this slip transposing method, in holding the slip by suction, at the time of lowering of the suction nozzle, the forcing means is compressed under the predetermined pressure from the tip suction port and the suction nozzle recedes to the rear end side, by which it is possible to absorb the displacement of the supposed stopping position of the suction nozzle. Also, at the time when the slip, being held under suction, is moved to the culture medium side and placed on the culture medium, and the nozzle tip holding the slip goes against the culture medium to give the predetermined pressure force, the suction nozzle is moved back to the rear end side. By this step, even in case of the movement beyond the position of contact with the culture medium surface, the movement is to be absorbed by the recession of the suction nozzle. Accordingly, without exerting the pressure force beyond necessity to the culture medium, spec to the culture medium surface that may be caused by the slip sucked by the suction nozzle is prevented. In addition, by the recession of the suction nozzle it becomes possible to absorb the irregularity of the culture medium surface.

The automatic slip transposing apparatus of the invention is an automatic slip transposing apparatus for taking out a predetermined number of slips from a plurality of slips supplied in a lump to the slip input portion and placing the respective slips on the predetermined positions of the culture medium, characterized by having a diversion part for dividing the slips sent out from the slip input portion in two rows in approximately the equal numbers, an array straight advance feeder whose base end is connected to the diversion part and for arraying approximately horizontally in two rows the slips sent from the slip input portion, a suction nozzle for suction-holding the slips on the array straight advance feeder by the tip suction port, and a pump for generating a suction force for the slip.

According to this constitution, it is possible to have the slips branched into two rows by the diversion part, array the slips in two rows approximately horizontally by the array straight advance feeder, and suction-hold the predetermined numbers of slips arrayed on the array straight advance feeder to transpose those slips on the respective predetermined positions on the culture medium.

The automatic slip transposing apparatus of the invention is an automatic slip transposing apparatus for taking out a predetermined number of slips from a plurality of slips supplied in a lump to the slip input portion and placing the respective slips on the predetermined positions of the culture medium, characterized by having a diversion part for dividing the slips sent from the slip input portion into two rows in approximately even numbers by a discharge channel provided by extension on the slip input portion and a branch channel for branching the discharge channel into two rows by step differences, and an array straight advance feeder for arraying the slips approximately horizontally in forward order in two rows by stopping the slips sent from the slip input portion at the tip. The base end of the feeder is connected to the branch channel of the diversion part.

According to this constitution, the slips sent from the slip input portion are arrayed in two rows in forward order. And, because a couple of slips on the same position are taken out from the respective rows of the array straight advance feeders, it becomes possible for the four slips to be transposed on the diagonal line of a square. By this step, it becomes possible for the four slips on the diagonal lines, which had so far been transposed manually to be automatically transposed in a simple structure and in high positional precision.

In the above-mentioned constitution, the diversion part may have a constitution provided with a baffle guide erected at approximately the central part of the discharge channel for dividing a plurality of slips sent from the slip input portion into approximately half and appropriate to the respective parts of the branch channel.

In this case, the slips sent to the discharge channel from the slip input portion are divided into approximately half and half by the baffle guide, and it does not occur for the slips to be supplied excessively to either one row in the array straight advance feeder. By this step, the slips charged in a lump are divided into the two branched channels respectively, and are evenly appropriated to the respective rows of the array straight advance feeder.

Alternatively, the apparatus may have a constitution comprising a first height control plate formed on the high position side of the branch channel and designed to pass only the slip on the lowest position out of the plural slips transposed in the upper and the lower layers and cause the slips other than in the lowest position to fall down to the low position side of the branch channel, and a second height control plate formed on the other side of the branch channel and designed to pass only the slip on the lowest position out of the plural slips transposed in the upper and the lower layers and cause the slips lying on the position higher than on the lowest position to fall down to the slip input portion.

By adopting such a constitution, the slips lying on the position other than on the lowest position sent in layers to one side of the branch channel are caused to fall down to the other side of the branch channel by the first height control plate. And, the slips lying on the position other than on the lowest position sent in layers to the other side of the branch channel are caused to fall down to the slip input portion by the second height control plate. By this step, the non-overlapped, arrayed slips are sent into the array straight advance feeder, and the surplus slips lying in layers in the respective parts of the branched channel are returned to the slip input portion and re-transposed.

The automatic slip transposing apparatus of the invention is an automatic slip transposing apparatus for taking out a predetermined number of slips from a plurality of slips of the slip supply portion and placing the respective slips on the respective predetermined positions of the culture medium, characterized by having a suction nozzle for adsorbing the predetermined number of slips and transposing them to the culture medium, a pump for generating slip suction force, a hose whose one end is communicated with the suction nozzle and the other end is branched to two ways, with the branched one part being connected to a pump and the branched other part being opened to the atmosphere and having elasticity, and a pinch valve for selectively clamping either side of the branched channel of the other end of the hose to block.

According to this constitution, due to the selective blocking of one or the other branched part of the other end of the hose by the vacuum pinch valve, the vacuum suction of the slip during its suction-holding and the introduction of the atmosphere at the time of the release of holding of the slip can be realized by a simple structure.

In the above-mentioned constitution, the constitution may be such that the slip supply portion carries the slips by arraying in a line in the groove to the suction position of the suction nozzle, that it has an array straight advance feeder having at least one groove, and a concave part is formed along the arrayed straight advance feeder at approximately the central part of the groove bottom surface of the array straight advance feeder and at least in the vicinity of the suction position of the suction nozzle.

In this case, when the slip is held by suction with the suction nozzle, the air passing through the slip is sucked from the concave part, and the inhalation from the slip side source is reduced. By this step, misfeeding of simultaneously adsorbing the adjacent slips in the groove is prevented.

The constitution may be such that provided with a pressure detection sensor for detecting the air pressure in the hose at the time of the suction holding of the slips.

By making the constitution as above, the vacuum in the hose is detected by the pressure sensor during the holding by suction of the slip, by which the pressure variation in the hose at the time when the suction nozzle failed to adsorb the slip is made detectable, and the holding mistake of the slip by the adsorbing nozzle is made detectable.

Further, the automatic slip transposing apparatus of the invention is an automatic slip transposing apparatus for taking out a predetermined number of slips from a plurality of slips of the slip supply portion and placing the respective slips on the respective predetermined positions of the culture medium, characterized by having a suction nozzle for suction-holding the slips on the slip supply portion by the tip suction port for inhaling the atmosphere, a nozzle block which is movable between the slip supply portion side and the culture medium side and which supports the adsorbing nozzle, a driving cylinder for moving the nozzle block in a manner that the suction port of the suction nozzle is movable forward and backward to the slip in the slip supply portion, and to the culture medium in the slip supply portion and to the culture medium in the culture medium side, and forcing means for forcing the suction nozzle in the tip suction port to the nozzle block and being possible to compress by the predetermined pressing force from the tip.

According to this constitution, in the ordinary time, the suction nozzle is forced to the nozzle block by the forcing means and disposed by biasing toward the tip end side. Accordingly, when the nozzle block holding the slip by suction is moved by the driving cylinder and the tip holding the slip goes against the culture medium to have the predetermined pressure force, the suction nozzle is moved back to the rear end side. In other words, even in case of moving to a level higher than the position of contact with the culture medium surface, the movement is to be absorbed by the retreat of the suction nozzle. By this step, no pressure force more than necessary is exerted to the culture medium and the spec to the culture medium surface by the suction nozzle is prevented. In addition, by the retreat of the suction nozzle, it becomes possible to absorb the irregularity of the culture medium surface. Furthermore, as the tip of the suction nozzle can be brought into contact with the culture medium in the range of movement in the axial line direction, the movement errors of the driving cylinder are absorbed, and it becomes possible to make the position control of the driving cylinder in relatively low precision.

### Brief Description of Drawings

Fig. 1 is a perspective view of the automatic slip transposing apparatus according to the invention taken from the lateral side.
Fig. 2 is a side view of the automatic slip transposing apparatus shown in Fig. 1.
Fig. 3 is an enlarged view of the essential part of Fig. 2.
Fig. 4 is a perspective view taken along the A-A section of Fig. 2 with a part omitted.
Fig. 5 is a perspective view of the diversion part.
Fig. 6 is a perspective view of the height control part.
Fig. 7 is a circuit diagram of the air pressure system for carrying out distribution, changeover, and detection of the air pressure.
Fig. 8 is an enlarged view of the essential part of the pinch valve.
Fig. 9 is a perspective view of an array straight advance feeder with the concave part.
Figs. 10(a) and (b) are illustrative views of the action of the suction nozzle forced by the forcing means.
Fig. 11 is a conceptual view representing the process for the lump charged disks until they are transposed onto the laboratory dish.
Fig. 12 is a perspective view of a diversion part of the other embodiment of the array structure part.
Fig. 13 is a perspective view of the laboratory dish in which the disks are transposed to the predetermined position.

### Mode for Carrying out the Invention

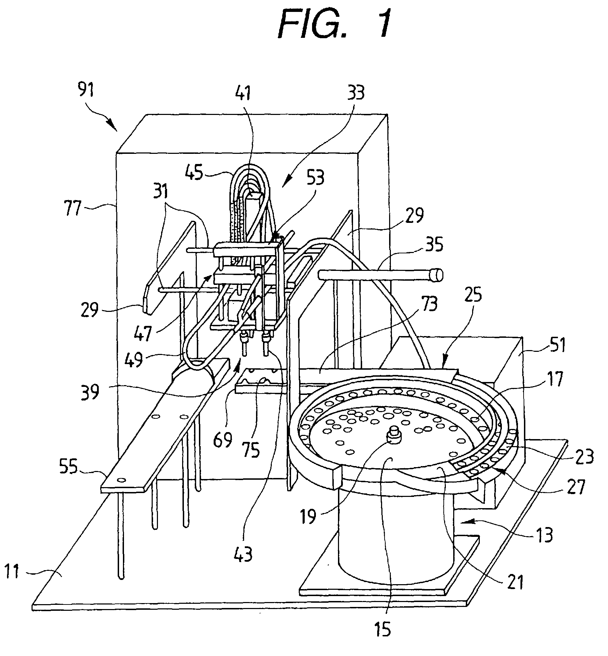

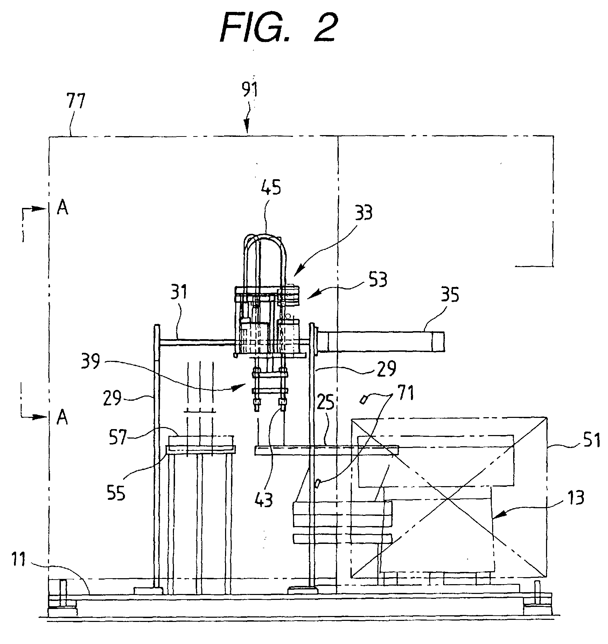

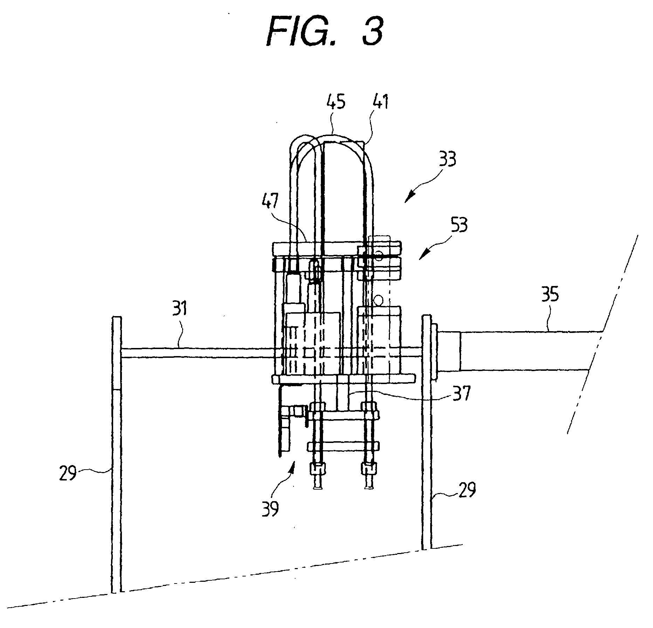

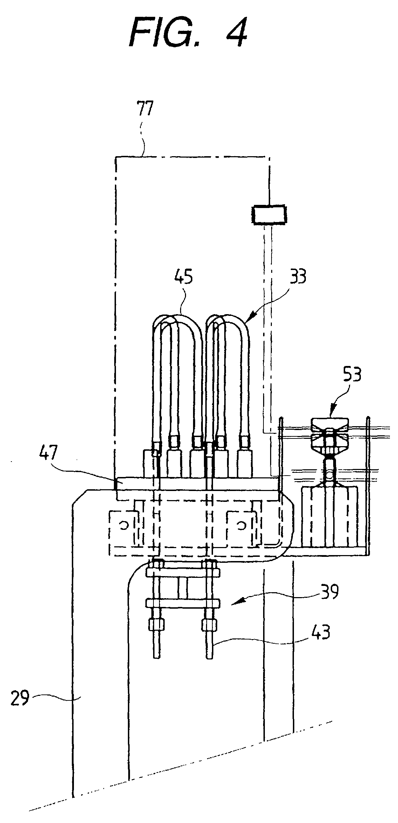

Hereinafter, preferred embodiments of the present invention are explained in detail with reference to the accompanying drawings. Fig. 1 is a perspective view of the automatic slip transposing apparatus according to the invention taken from the lateral side. Fig. 2 is a side view of the automatic slip transposing apparatus shown in Fig. 1. Fig. 3 is an enlarged view of the essential part of Fig. 2. Fig. 4 is a perspective view taken along the A-A section of Fig. 2 with a part omitted. For convenience of the illustration, explanation is given on assumption of the right/left direction in Fig. 3 to be the X-axis direction and the up/down direction in Fig. 3 to be the Z-axis direction.

On the base plate 11 there is installed a disk supply feeder 13 for supplying the slips (hereinafter to be called alias "disk"). The upper part of the disk supply feeder 13 is formed to be a slip input portion (ball part) 15. On the ball part 15 a plurality of disks 17 are inputted in a lump. The bottom plate of the ball part 15 is movable back and forth in a circumferential direction by a minor amount centering on the center axis 19 while vibrating in the up/down directions. Accordingly, a plurality of disks 17 inputted in a lump in the ball part 15 are individually separated and moved in rotation in one direction (anti-clockwise direction in Fig. 1) while being brought toward outside the radial direction of the ball part 15.

On the outer periphery of the ball part 15 a discharge channel 21 is extensively provided. Accordingly, the disk 17 brought toward the outer peripheral side of the ball part 15 and moved in rotation is discharged outside from the discharge channel 21. The discharge channel 21 is branched into two parts by a step difference to become the branch channels 23. The discharge channel 21 and the branch channels 23 constitute a diversion part 27 to be described later. The branch channels 23 are connected respectively to the array straight advance feeder 25. That is to say the disk 17 sent from the ball part 15 reaches the array straight advance feeder 25 through the diversion part 27. Besides, there is a constitution that the two rows of the disk 17 come to be approximately the horizontal positions in the array straight advance feeder 25. By these disk supply feeder 13 and array straight advance feeder 25 a slip (disk) supply part is constituted.

On the base plate 11 a couple of stays 29, 29 are erected. Between these stays 29, 29 a couple of rail rods 31, 31 are provided. With the parallel direction of these rail rods 31, 31 taken as X-axis direction, a slide block 33 is fitted to the rail rods 31, 31 in freely slidable manner in an X-axis direction.

On one part of the stays 29, 29, an X-axis driving cylinder 35 is fixedly provided, and the driving shaft of the X-axis driving cylinder 35 is fixed to the slide block 33. In other words, the slide block 33 is moved in the X-axis direction along the rail rods 31, 31 by the driving of the X-axis driving cylinder 35.

To the lower part of the slide block 33, a nozzle block 39 is freely movably mounted in a Z-axis direction, which is a vertical downward direction, through a vertical shaft 37. To the upper part of the slide block 33, there is fixed a driving cylinder 41 for the vertical shaft 37, which is a Z-axis. To this vertical shaft 37, a nozzle block 39 is fixed. Namely, the nozzle block 39 is moved in the Z-axis direction by the driving of the Z-axis driving cylinder 41.

To the lower part of the nozzle block 39, there are provided four suction nozzles 43 in the Z-axis direction in the square diagonal positions (e.g., 36 X 36 mm) and at the same height. These suction nozzles 43 have the openings for suction at the lower ends, details of which will be described later. In case the size of the disk 17 is 6 mm in diameter, the suction nozzle 43 is set to be about 2 mm in inner diameter, and about 4 mm in outer diameter. This caliber is not limited to these numerical values insofar as it is the size capable of sucking the disk.

To the respective suction nozzles 43 the hoses 45 are connected, and the hoses 45 are connected to the manifold 47. To the manifold 47 a main hose 49 is connected, and the main hose 49 is connected to the vacuum pump 51. To the main hose 49 between the manifold 47 and the vacuum pump 51 a vacuum pinch valve 53 is provided. The vacuum pinch valve 53 is made so as to allow the main hose 49 to be selected to the vacuum pump 51 or to the atmosphere side.

On the other hand, on the base plate 11 a laboratory dish setting part 55 is provided, and on the top face of the laboratory dish setting part 55 there is disposed a stopper capable of setting the laboratory dish 57 on the predetermined position for transposing the disk 17. Above the laboratory dish 57 set to the laboratory dish setting part 55, a slide block 33 can be disposed by sliding movement.

The array straight advance feeder 25 has two rows of the carrier channels for carrying the disks 17 arrayed in a row. These carrier channels are connected respectively to the branch channels 23. At the tip of the array straight advance feeder 25 a stopper 69 is provided. This stopper 69 controls the movement of the disk 17 at the tip arrayed on the array straight advance feeder 25. Further, on the top face of the array straight advance feeder 25 a cover 73 is provided.

That is to say, in the array straight advance feeder 25, the topmost disk 17 is checked up so as to bring the succeeding disks forward in order. By the provision of a cover 73, the adjacent disks 17 are prevented from overlapping, and the disks are advanced forward in order. Therefore, when the disks 17 are taken out from the topmost part, the space formed is to be filled by the succeeding disks 17. And, the cover 73 is provided with the four taking out holes 75 (See Fig. 1). The taking out holes 75 are provided on the positions from which a couple of disks 17 on the same positions can be taken out from the respective rows of the array straight advance feeder 25.

By providing the cover 73 and the array straight advance feeder 25 with some spaces and constituting the groove for moving the disk 17 to be visible, any choking up of the disks 17 can be confirmed through the space, and the choking up state can be rescinded.

Though the detailed description will follow, a concave part is formed along the array straight advance feeder 25 at approximately the central part of the bottom surface of the groove in which the disks 17 of the array straight advance feeder 25 are aligned.

In the upper and lower parts of the array straight advance feeder 25 there are provided the disk detection sensors 71 (See Fig. 2) for detecting the presence or not of the disks 17 in array state. The base plate 11 is provided with a controller 77. The controller 77 is electrically connected to a disk supply feeder 13, the X-axis driving cylinder 35, the Z-axis driving cylinder 41, the vacuum pump 51, the disk detection sensor 71, and the like.

Next, the array structure of the disk 17 is explained.

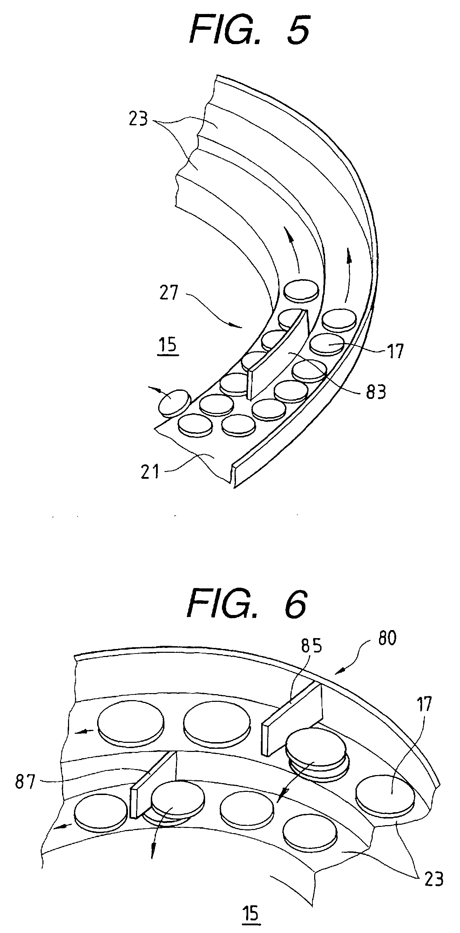

Fig. 5 is a perspective view of the diversion part, and Fig. 6 is a perspective view of the height control part.

The disks 17, which are sent to the discharge channel 21 are divided into approximately half-and-half by the diversion part 27, and they are prevented from being piled by the height control part 80. The diversion part 27 includes a discharge channel 21, a branch channel 23, and a baffle guide 83. The baffle guide 83 is erected at approximately the central part of the discharge channel 21. A plurality of disks 17 sent from the ball part 15 are divided into approximately half and half and put to the respective parts in the branch channels 23.

In this embodiment, as shown in Fig. 5, the discharge channel 21 is curved, and at approximately the central part a baffle guide 83 is provided upright. In the area between the baffle guide 83 and the outside of the discharge channel 21, and the area between the baffle guide 83 and the inside of the discharge channel 21, a passage width corresponding to approximately the diameter of the disk 17 is secured. Also, it is so designed that the disk 17 can fall down from the edge inside the discharge channel 21 to the ball part 15.

Accordingly, in case the disks 17 sent from the discharge channel 21 are voluminous, the disks 17 for one row portion are allocated to the outside of the discharge channel 21. For the remaining disks 17, the disks 17 for one row portion are allocated to the inside of the discharge channel 21. Further, the excessive disks 17 that have not been arrayed inside the discharge channel 21 are to fall down from the edge inside the discharge channel 21 to the ball part 15. Further, in case the disks to be sent from the discharge channel 21 are few in number, the disks 17 sent from the discharge channel 21 are allocated to nearly half and half and sent to the respective branch channels 23. In other words, irrespective of the case where the disks 17 to be sent are large or small in number, the disks 17 are to be divided evenly.

The height control part 80 includes a first height control plate 85 provided on one side of the branch channel 23 and a second height control plate 87 provided on the other side of the branch channel 23. The first height control plate 85 allows to pass only the lowermost disk 17 out of the plural disks 17 piled up and down in layers in one branch channel 23, and causes the disks 17 lying above the lowermost level to fall down to the other part of the branch channel 23. The second height control plate 87 allows to pass only the lowermost disk 17 out of the plural disks 17 piled up and down in layers in the other branch channel 23, and causes the disks 17 lying above the lowermost level to fall down to the ball part 15.

Next, the suction holding structure of the disk 17 is explained.

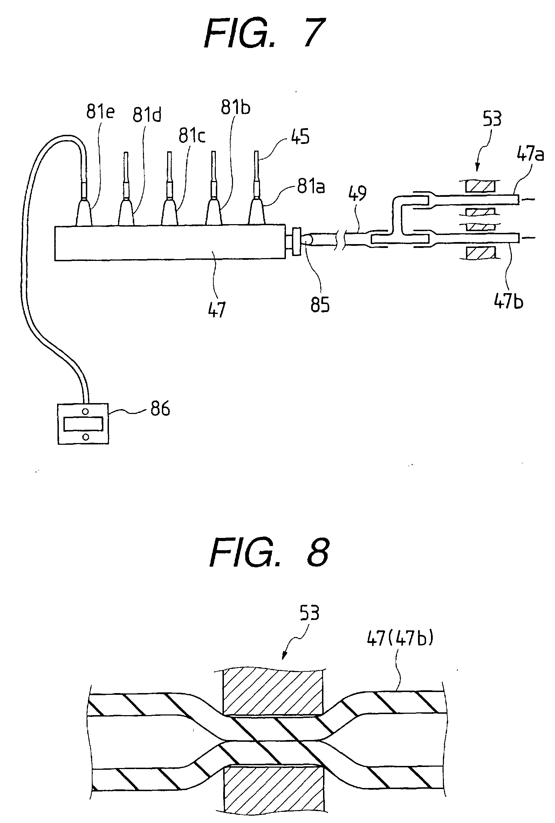

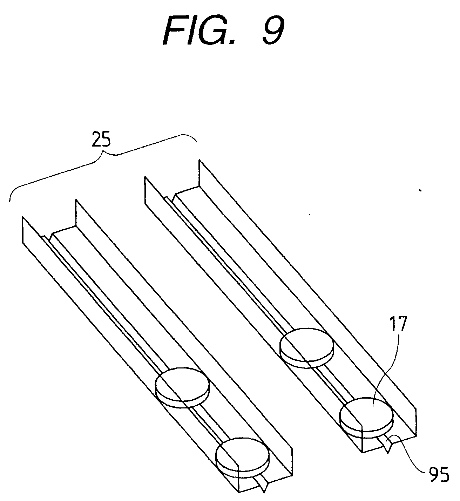

Fig. 7 is a circuit diagram of an air pressure system for carrying out distribution, changeover, and detection of the air pressure. Fig. 8 is an enlarged view of the essential part of the pinch valve. Fig. 9 is a perspective view of an array straight advance feeder with the concave part.

As shown in Fig. 7, the manifold 47 includes five branch connection ports 81a, 81b, 81c, 81d, and 81e, of which the branch connection ports 81a-81d are connected to the suction nozzle 43. The branch connection port 81e is connected to the air pressure detection sensor 86. All these branch connection ports 81a-81e are connected to the main hose 49 through the main connection port 85.

Here, the air pressure detection sensor 86 is electrically connected to the controller 77, and detects the pressures in the air pressure circuit and sends the detection signals to the controller 77. The controller 77 is to carry out confirmation of the disk suction holding motion by the detection signal sent from the air pressure detection sensor 86. That is to say, in the state of adsorption holding of the disk 17 by the suction nozzle 43, if one of the four suction nozzles 43 does not adsorb the disk 17, the pressure in the air circuit remains to be kept to a level near the atmospheric pressure without reaching the predetermined negative pressure. Namely, the controller 77 can detect whether the suction holding of the disk 17 is surely performed or not, by detecting the pressure in the air pressure circuit during the disk holding by the air pressure detection sensor 86.

The main hose 49 has one end communicated with the suction nozzle 43 through the manifold 47 and the other end branched to two ways to make the branched hoses 47a, 47b. In the main hose 49, the branched one part 47a is connected to the vacuum pump 51, and the other part 47b is opened to the atmosphere. The branched hoses 47a, 47b are formed of the material having elasticity (e.g., silicon rubber). As these branched hoses 47a, 47b can be selectively blocked (See Fig. 8), either the branched one part 47a or the other part 47b can be closed.

Accordingly, the air pressure circuit comes to have negative pressure because the branch hose 47b opened to the atmosphere is blocked by the pinch valve 53 and the blockade of the branched hose 47a is released. Also, the atmospheric pressure is introduced by the release of the blockade of the branched hose 47b and the blockade of the branched hose 47a connected to the vacuum pump 51.

The disks 17 sent from the ball part 15 are arrayed in the array straight advance feeder 25 and carried as described above. At approximately the central part of the groove bottom part in which the disks 17 of the array straight advance feeder 25 are arrayed, there is formed as shown in Fig. 9 a groove form concave part 95 provided by extension along the array straight advance feeder 25. The array straight advance feeder 25 can give influx of air in the lower face of the disk 17 because of the presence of the concave part 95. That is to say, the air which passes through the disk 17 and is sucked in at the time of the suction holding of the top face of the disk 17 having air permeability is to go through the concave part 95. Though it is defined that the concave part 95 is to be provided in extension along the groove of the array straight advance feeder 25, any other constitution may be adopted if the flow channel of air that is sucked in by passing through the disk 17 is secured, by providing a concave part only in the vicinity of the taking out hole 75 position in which at least the suction nozzle 43 is receivable.

Next, explanation is given on the forcing structure part for the suction nozzle 43.

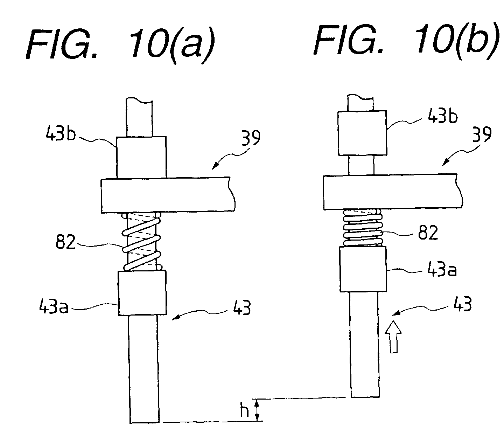

Figs. 10(a) and (b) are illustrative views of the action of the suction nozzle forced by the forcing means.

The suction nozzle 43 is formed linearly, and is capable of suction holding of the disk 17 by inhaling air from the tip. This suction nozzle 43 is supported, as described above, by the sliding block 33 and the nozzle block 39 in the manner movable in the Z-axial line direction. In this embodiment, the suction nozzle 43 is supported to have its axial line in a vertical direction. Between the first stopper 43a mounted to the suction nozzle 43 and the nozzle block 39, there is provided a forcing means (e.g., a coil spring 82). The coil spring 82 forces the suction nozzle 43 toward the tip end side in biased state, as shown in Fig. 10(a). And, as the second stopper 43b fitted to the suction nozzle 43 comes into contact with the nozzle block 39 to stop the force of the coil spring 82, the initial position of the suction nozzle 43 is supported.

Next, the action of the automatic slip transposing apparatus 91 constituted as above is explained.

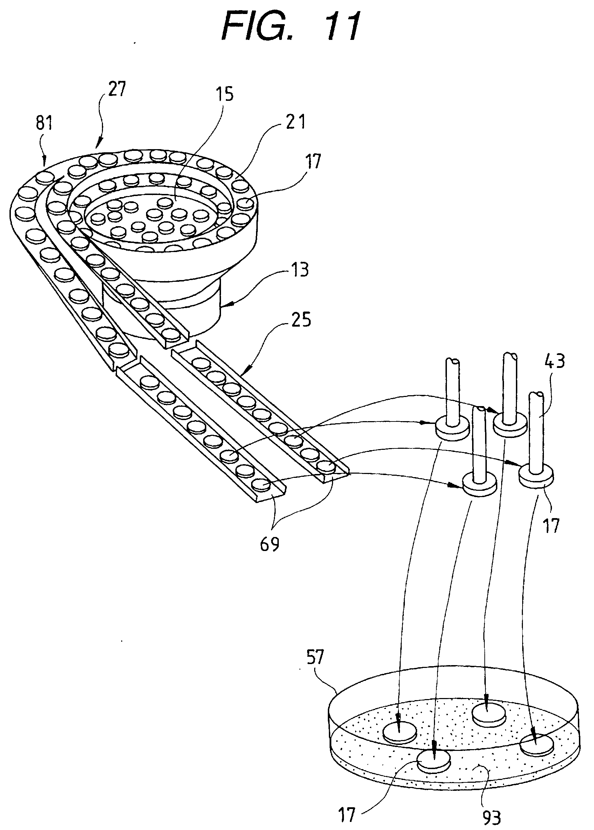

Fig. 11 is a conceptual view representing the process for the lump charged disks until they are transposed onto the laboratory dish.

In the first place, as a preparation for the operation, the disk 17 is charged into the ball part 15 of the disk supply feeder 13 to reinstate the apparatus to the original point.

The disks 17 in the ball part 15 are sent to the diversion part 27 from the discharge channel 21 while being unfastened under vibration. The disks 17, which reached the diversion part 27, are classified by the baffle guide 83 and sent to the branch channel 23 in a row (See Figs. 5 and 6).

In this case, the overlapped disks 17 are controlled to a sheet by the first height control plate 85 and the second height control plate 87 in the respective branch channels 23. The disks 17, which became surplus are dropped to the ball part 15, and again sent out from the discharge channel 21.

The disks 17 sent into the branch channel 23 go into the array straight advance feeder 25 in two rows, and are put forward by the tip end stopper 69. When the disks 17 are set toward the front part to the predetermined position of the array straight advance feeder 25, the disk detection sensor 71 detects the disks 17, and the driving of the disk supply feeder 13 is stopped (See Fig. 2). By this action, the excessive supply of the disks 17 is prevented. When the disks 17 are supplied and arrayed to this position, the reinstatement to the original point of the apparatus is to be terminated. Further, during the performance of a series of motions, the suction nozzle 43 is also subjected to the motion for reinstatement to the original point.

Here, a laboratory dish 57 in which the agar plate culture medium 93 is formed, with a lid removed, is placed on the laboratory dish setting part 55 (See Figs. 1 and 2), and the driving switch of the controller 77 is depressed, by which the four suction nozzles 43 descends by the predetermined amount to come into light contact with the top face of the disks 17 in the array straight advance feeder 25 through the taking out holes 75. The distance of descent of the suction nozzle 43 is set to a degree that the suction nozzle 43 comes into contact with the disks 17 and the coil spring 82 is slightly pushed in (See Figs. 10(a) and (b)).

Next, when the vacuum pinch valve 53 is operated to block the branch hose 47b opened to the atmosphere and open the branch hose 47a connected to the vacuum pump 51, a vacuum pressure is exerted to the suction nozzle 43, and the disks 17 are suction held by the suction nozzle 43. The controller 77 confirms that the disks 17 could be surely adsorbed by the air pressure detection sensor 86, after which the controller 77 drives the Z-axis driving cylinder 41 to cause the suction nozzle 43 to rise, and takes out the disks 17 from the taking out hole 75 of the array straight advance feeder 25. If, in this case, the air pressure detected by the air pressure detection sensor 86 does not reach the predetermined negative pressure, then the controller 77 judges to be defective adsorption and sends out the stoppage signal of the device.

Next, when the X-axis driving cylinder 35 is driven, the slide block 33 is moved onto the laboratory dish 57 and the Z-axis driving cylinder 41 is driven again, by which the disks 17 held by the suction nozzle 43 are set on the agar plate culture medium 93 in the laboratory dish 57. Continuously, the Z-axis cylinder 41 is driven to lower the suction nozzle 43 and allow the disks 17 to come into contact with the agar plate culture medium 93.

In this case, it is suitable that, in consideration of the shift amount error of the Z-axis driving cylinder 41, or the irregularity of the agar plate culture medium 93, the supposed position of stoppage of the tip of the suction nozzle 43 is set to a degree for the tip to pass over the surface of the agar plate culture medium 93 to some extent.
At this time, the suction nozzle 43 is, as shown in Fig. 10 (b), pushed in to the rear end side (upper side) resisting the coil spring 82. That is to say, the disagreement between the surface position of the agar plate culture medium 93 and the supposed position of stoppage of the tip of the suction nozzle 43 is to be absorbed by the pushing-in of the suction nozzle 43. By this step, the tip of the suction nozzle 43 is to stop at all times in contact with the surface of the agar plate medium 93 through the disk 17, so as not to exert the load beyond necessity to the surface of the agar plate medium 93.

By operation again of the vacuum pinch valve 53 under this state, the branch hose 47a opened to the atmosphere is opened and the branch hose 47b connected to the vacuum pump 51 is blocked, by which the atmospheric pressure is introduced to the suction nozzle 43. By this step, under impact of the return to the atmosphere, the slips held by suction are to be surely released from the suction nozzle. Accordingly, the disks 17 released from suction holding are simultaneously transposed to the predetermined position on the agar plate culture medium 93.

Next, after detection by the air pressure detection sensor 86 that the contents in the hose have come to the atmospheric pressure, namely, that the transposition of the disks 17 has completed, the suction nozzle 43 is elevated by the Z-axis driving cylinder 41, and again returned to the adsorbing position of the disks 17 to assume the waiting condition.

The laboratory dish 57 on which the disks 17 are transposed is manually taken out from the laboratory dish setting part 55 and the lid is closed, by which the disk transposing work is completed.

As described above, according to this automatic slip transposing apparatus 91, the disks 17 sent from the ball part 15 to the discharge channel 21 are divided into approximately half and half by the baffle guide 83, and it does not occur for the disks 17 to be excessively supplied to either one row only of the array straight advance feeder 25. By this, the disks 17 charged in a lump are distributed to the respective parts of the branch channels 23 branched into two parts and evenly divided into the respective rows of the array straight advance feeder 25.

Further, the disks 17 lying on the position other than on the lowest position sent in layers to one side of the branch channels 23 are caused to fall down to the other side of the branched channels 23 by the first height control plate 85. And, the disks 17 lying on the position higher than on the lowest position sent in layers to the other side of the branch channel 23 are caused to fall down to the ball part 15 by the second height control plate 87. By this step, the non-overlapped, arrayed disks 17 are sent into the array straight advance feeder 25, and the surplus disks 17 lying in layers in the respective parts of the branch channels 23 are returned to the ball part 15 and re-transposed.

As a result, the disks 17 sent from the ball part 15 are arrayed in two rows in forward order at the array straight advance feeder 25. And, because a couple of disks 17 on the same position are taken out from the respective rows of the array straight advance feeder 25, it becomes possible for the four disks 17 to be transposed on the diagonal line of a square. By this step, it becomes possible for the four disks 17 on the diagonal lines, which had so far been transposed manually to be automatically transposed in a simple structure and in high positional precision.

According to this automatic slip transposing apparatus 91, because the branched hose 47b opened to the atmosphere is blocked, the space inside the hose comes to be of a negative pressure and the disks 17 are held by suction by the air sucked from the suction nozzle 43. On the other hand, the branch hose 47b is opened and the branch hose 47a is blocked, thereby introducing the air pressure into the hose, and the disk 17 held by suction is surely separated from the suction nozzle 43.

Further, because the branch hoses 47a, 47b are selectively clamped by the vacuum pinch valve 53, the vacuum suction of the disks 17 during suction holding and the introduction of air at the time of the release of holding of the disks 17 can be realized by a simple structure, without necessitating a complicated and precise valve structure.

Also, when the disk 17 is held by suction with the suction nozzle 43, the air passing through the disk 17 is sucked from the concave part 95, and the inhalation from the side surface of the disk is reduced. By this step, the adjacent disks 17 are prevented from being adsorbed.

Furthermore, as the vacuum in the hose is detected by the air pressure detection sensor 86 during the holding by suction of the disks 17, the pressure variation in the hose at the time when the suction nozzle 43 failed to adsorb the disk 17 is made detectable, and the holding mistake of the disk 17 by the adsorbing nozzle 43 is made detectable.

Further, according to the automatic slip transposing apparatus 91 of the invention, the suction nozzle 43 is forced by a coil spring 82 and biased to the tip side and held by the nozzle block 39. Accordingly, when a suction nozzle 43 holding by adsorbing the disks 17 goes against the Z-axis driven agar plate culture medium 93, the suction nozzle 43 is pushed in to the rear end side.
In other words, even when going against the agar plate medium 93 and the supposed stopping position of the suction nozzle 43 is higher than the surface position of the agar plate medium 93, the movement above the surface position is to be absorbed by the pushing-in of the suction nozzle 43.
By this step, no pressure force more than necessary is exerted to the agar plate culture medium 93, and the spec to the surface of the agar plate culture medium 93 by the suction nozzle 43 is prevented. And, because the suction nozzle 43 has a mechanism possible to make retreat, it becomes possible to absorb the irregularity or inclination of the agar plate culture medium 93.

Furthermore, as the tip of the suction nozzle 43 can be brought into contact with the agar plate culture medium 93 in an optional position in the range of movement h in the axial line direction (See Figs. 10(a) and (b)), the movement errors of the Z-axis driving cylinder 41 are absorbed, and it becomes possible to make the position control of the Z-axis driving cylinder 41 in relatively low precision.

Further, by abruptly introducing the air pressure at the time of transposing the disks 17 onto the agar plate culture medium 93 from the suction nozzle 43, the disks 17 held by suction on the suction nozzle 43 are instantaneously blown off, and the disks 17 released from suction holding are surely transposed to the predetermined position on the agar plate culture medium 93.

Next, explanation is given on other embodiment of the array structural part of the disks 17.

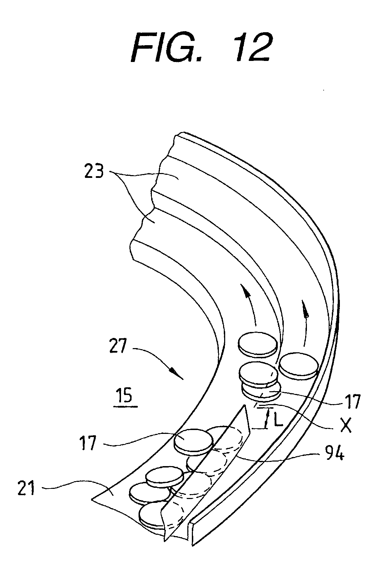

Fig. 12 is a perspective view of a diversion part of the other embodiment of the array structure part. With respect to the height control part, the constitution is similar to that of Fig. 6.

The disks 17, which are sent to the discharge channel 21 are divided into approximately half-and-half by the diversion part 27, and they are prevented from being piled by the height, control part 80. The diversion part 27 includes a discharge channel 21, a branch channel 23, and a baffle guide 94. The two branch channels 23 connected from the discharge channel 21 have such constitution that the inside branch channel 23 is gradually lowered from the branch point X to give a step difference. The baffle guide 94 is fixed with a distance L on the upstream side of the branch point X. A plurality of disks 17 sent from the ball point 15 are divided into approximately half and half lots by the baffle guide 94 and the branch point X and appropriated to the respective parts of the branch channel 23. The distance L is preferably set to be approximately half the diameter of the disk 17. Furthermore, the tip on the downstream side of the baffle guide 94 is disposed toward the branch channel 23 lying outside the branch point X.

In this embodiment, the discharge channel 21 is curved, and the tip part on the upstream side of the baffle guide 94 is disposed in contact with the sidewall. Therefore, when the disks 17 sent in the discharge channel 21 of the ball part 15 are moved along the topmost side wall of the ball part 15 and reach the baffle guide 94 position as in Fig. 12, they become movable along it.

As the disks 17 are arranged so as to reach the branch point X at the distance L of approximately half the diameter of the disk 17 from the tip on the downstream side of the baffle guide 94, the disks 17 that have come to be sent are naturally divided into the inside and the outside of the branch channel 23 under delicate balance. Accordingly, depending on the ways of taking the tip part on the upstream side of the baffle guide 94, the proportion of diversion can be set to a certain extent.

In this manner, in this embodiment, irrespective of the amount of the disks 17 to be sent from the discharge channel 21, it becomes possible to distribute the disks in the desired proportion. Accordingly, even in the condition where the disks 17 in the ball part 15 become nil, distribution in the desired proportion can be made up to the last disk 17. Namely, irrespective of whether the amount of the disks to be sent is large or small, the disks 17 are evenly distributed.

Next, the results of working the present invention and confirming the effects thereof are shown below.

Here, explanation is given on the results of survey on the working duration of the disk process in the case of the manual work and the case of using the automatic slip transposing apparatus according to the invention.

### 1. In case of manual work (personnel: 2 workers)

### (Working conditions)

Laboratory dish : 100
Disk : 4/laboratory dish
Repetition cycle : 3 times

### (Survey results)

| Cycle No. | Work duration | Average time required per laboratory dish |
|---|---|---|
| 1. | 16 min. 28 sec. | |
| 2. | 15 min. 18 sec. | Approx. 9.5 seconds |
| 3. | 15 min. 08 sec. | |

### 2. In case of manual work (personnel: 1)

### (Working conditions)

laboratory dish : 100
Disk : 4/laboratory dish
Repetition cycle: 3 times

### (Survey results)

| Cycle No. | Work duration | Average time required per laboratory dish |
|---|---|---|
| 1. | 18 min. 29 sec. | |
| 2. | 17 min. 13 sec. | Approx. 10.3 seconds |
| 3. | 15 min. 45 sec. | |

### 3. In case of using automatic slip transposing apparatus:

### (Working conditions)

laboratory dish : 100
Disk : 4/laboratory dish

### (Survey results)

Average time required
per laboratory dish
Approx. 8 seconds

As apparent from the results, in case of using the automatic slip transposing apparatus of the invention, it has been known that the time curtailment by 1.5 to 2.3 seconds per laboratory dish is practicable.

### Industrial Applicability

As apparent from the explanation, in the automatic slip transposing apparatus of the invention, the following advantageous points can be noted.
(1) By classifying the slips sent from the slip input portion in two rows approximately evenly, arraying the slips forward approximately horizontally in two rows, and taking out the predetermined number of the arrayed slips, the respective slips can be placed simultaneously on the predetermined positions of the culture medium.
(2) By making continuous suction from the suction nozzle, the air-permeable slips can be surely held. Also, because the suction holding is released by introducing the atmospheric pressure, the slips under suction holding state can be surely transposed by the impact at the time of the introduction of the atmosphere.
(3) Due to the provision of an air pressure detection sensor, it becomes possible to detect the pressure in the hose which varies in case of the failure of adsorption of the slips by the suction nozzle and detect the mistake of holding the slips by the suction nozzle. As a result, the mistake of transposing the slips can be prevented.
(4) In case of the suction holding of the slips, displacement of the supposed position of stoppage at the tip of the suction nozzle can be absorbed, and in case of placing the slips on the culture medium, because no pressing force beyond necessity is given to the culture medium, spec on the culture medium by the suction nozzle can be prevented, and irregularity on the culture medium surface can be absorbed. In addition, position control of the driving cylinder can be made in relatively low precision.
(5) By taking out a couple of slips on the same position from the respective array straight advance feeders arranged in parallel, the four slips can be simultaneously arranged on the diagonal lines of the square. Consequently, it becomes possible for the slips so far transposed manually to be automatically transposed in a simple structure.
(6) By preventing the excessive supply of the slips sent from the slip input portion only to the one side row of the array straight advance feeder, the slips can be evenly divided into two rows of the array straight advance feeder.
(7) It is possible to send non-piled, arrayed slips to the respective rows of the array straight advance feeder, and to return the overlapped excessive slips to the input part. As a result, it is possible to ensure takeout of the slips from the array straight advance feeder, and to elevate reliability of the automatic transposing motion of the slips.
(8) By selectively clamping the hose connected to the pump connected to the suction nozzle and the hose opened to the atmosphere, there can be obtained a choking-free, highly reliable valve structure in a simple construction without necessitating complicated and precise valve structure.
(9) When the slips on the array straight advance feeder is held by the suction nozzle, the air passing through the slips can be sucked up from the concave part lying along the array straight advance feeder, and misfeed of adsorbing the adjacent slips by inhalation of air from the side surface of the slip can be prevented.

## Claims

1. A slip transposing method comprising:
branching in two rows and nearly horizontally arraying slips (17) sent out from a slip input portion which supplies a plurality of slips (17) in a lump;
holding by suction the predetermined number of the arrayed slips (17) on each row by a suction nozzle (43) connected to a pump (51); and
transposing the slips (17) onto respective predetermined positions on a culture medium (93).

2. A slip transposing method comprising:
sending out a plurality of slips (17) sequentially from a slip input portion supplying the slips (17) in a lump to a discharge channel (21);
dividing the slips (17) approximately evenly into two rows by a step difference provided halfway in the discharge channel (21);
stopping the slips (17) at a tip, thereby arraying the slips (17) in forward order approximately horizontally in two rows;
taking out the predetermined number of the arrayed slips (17);
placing each of the slips (17) in a predetermined position of a culture medium (93).

3. A slip transposing method comprising:
communicably connecting an end of an elastic hose to a suction nozzle (43) for slip;
branching the other end of the elastic hose into two ways, and connecting a branched one part to a pump (51) and opening the other part to the atmosphere;
suction-holding slips (17) from a slip supply portion with a suction nozzle (43) by rendering an inside of the hose to a negative pressure by the pump (51) with blocking the hose on the side opened to the atmosphere; and
transposing the slips (17) onto the culture medium (93) from the slip supply portion by releasing the suction-holding of the slips (17) by the nozzle (43) with blocking the hose on the side connected to the pump (51) and opening the hose on the side opened to the atmosphere, thereby rendering the air inside the hose to an atmospheric pressure.

4. The slip transposing method according to claim 3, wherein, during the suction-holding of the slips (17), the air pressure in the hose is detected by an air pressure detection sensor (86), and if a detected value is higher than a predetermined air pressure, the suction is released.

5. A slip transposing method for taking out the predetermined number of slips out of a plurality of the slips (17) in a slip supply portion and transposing each of the slips (17) to a predetermined position on a culture medium (93), wherein
in suction-holding the slips (17) on the slip supply portion by a suction port at a tip of a suction nozzle (43) for sucking the air, and in placing the slips (17) on the culture medium (93) by moving them from the slip supply portion side to the culture medium side,
a forcing means (82) provided on the suction nozzle (43) and forcing in a direction of the suction port at the tip of the nozzle is compressed under a predetermined pressure force from the suction port at the tip at the time of lowering of the suction nozzle (43), thereby causing the suction nozzle (43) to recede to a rear end side.

6. An automatic slip transposing apparatus for taking out the predetermined number of slips from a plurality of the slips (17) supplied in a lump to a slip input portion and placing each of the slips (17) on a predetermined position of a culture medium (93), comprising:
a diversion part (27) for dividing the slips (17) sent out from the slip input portion into two rows in approximately an equal number;
an array straight advance feeder (25) whose base end is connected to said diversion part (27) and for arraying the slips (17) sent from the slip input portion approximately horizontally in two rows;
a suction nozzle (43) for suction-holding a predetermined number of the slips (17) on the array straight advance feeder (25) by the tip suction port; and
a pump (51) for generating a suction force to the slip (17).

7. An automatic slip transposing apparatus for taking out the predetermined number of slips from a plurality of the slips (17) supplied in a lump to a slip input portion and placing each of the slips (17) on a predetermined position of a culture medium (93), comprising:
a diversion part (27) for dividing the slips (17) sent from the slip input portion into two rows in approximately even numbers by a discharge channel (21) provided by extension on the slip input portion and a branch channel (23) for branching the discharge channel (21) into two rows by a step difference; and
an array straight advance feeder (25) for arraying the slips (17), whose base end is connected to the branch channel of the diversion part (27) and which is sent from the slip input portion, approximately horizontally in forward order in two rows by stopping at a tip end (69).

8. The automatic slip transposing apparatus according to claim 7, wherein the diversion part (27) is provided with baffle guides (83, 94) erected at approximately a central part of the discharge channel (21) for dividing the plurality of slips (17) sent from the slip input portion into approximately half and half and appropriating to the respective parts of the branch channel (23).

9. The automatic slip transposing apparatus according to claim 7 or 8, comprising:
a first height control plate (80) formed on a high position side of the branch channel (23) and designed to pass only the slip (17) on the lowest position out of the plurality of the slips (17) transposed in an upper layer and a lower layer and to cause the slips (17) other than in the lowest position to fall down to a low position side of the branch channel (23); and
a second height control plate (87) formed on the other side of the branch channel (23) and designed to pass only the slip (17) on the lowest position out of the plurality of slips (17) transposed in an upper layer and a lower layer and cause the slips (17) lying on the position higher than on the lowest position to fall down to the slip input portion.

10. An automatic slip transposing apparatus for taking out the predetermined number of slips (17) from a plurality of the slips (17) in a slip supply portion and placing each of slips (17) on a predetermined position of the culture medium (93), comprising:
a suction nozzle (43) for adsorbing the predetermined number of slips (17) and transposing the slips to a culture medium (93);
a pump (51) for generating a suction force to the slip (17);
a hose whose one end is communicated with the suction nozzle (43) and the other end is branched in two ways, with the branched one part being connected to the pump (51) and the branched other part being opened to an atmosphere and having elasticity; and
a pinch valve (53) to block by selectively clamping one side of the branched two ways of the other end of the hose.

11. The automatic slip transposing apparatus according to claim 10, wherein:
the slip supply portion has an array straight advance feeder (25) including at least one groove, arraying the slips (17) in a row in the groove, and carrying them to a suction position of the suction nozzle (43); and
a concave part (95) is formed along the array straight advance feeder (25) at approximately a central part of a groove bottom surface of the array straight advance feeder (25) and at least in the vicinity of the suction position of the suction nozzle (43).

12. The automatic slip transposing apparatus according to claim 10 or 11, wherein an air pressure detection sensor (86) is provided for detecting an air pressure in the hose at the time of suction-holding the slips (17).

13. An automatic slip transposing apparatus for taking out the predetermined number of slips (17) from a plurality of the slips (17) in a slip supply portion and placing each of slips (17) on a predetermined position of the culture medium (93), comprising:
a suction nozzle (43) for suction-holding the slips (17) on the slip supply portion by a tip suction port for inhaling the atmosphere,
a nozzle block (39) movable between a slip supply portion side and a culture medium side and supporting the suction nozzle (43),
a driving cylinder (41) for moving the nozzle block (39) in a manner to make the suction port of the suction nozzle (43) movable forward and backward to the slip (17) in the slip supply portion and to the culture medium (93) in the culture medium side, and
forcing means (82) for forcing the suction nozzle (43) to the nozzle block (39) in a direction of the tip suction port and compressible by a predetermined pressing force from a tip thereof.
